# EUROPEAN PATENT APPLICATION

(11) **EP 3 892 738 A1**
(43) Date of publication of application: **13.10.2021**
(21) Application number: 20315148.5
(22) Date of filing: 10.04.2020
(51) Int. Cl.: C12Q 1/6883, A61K 31/00, A61P 25/00

(54) **G-PROTEIN-GATED-K+ CHANNEL-MEDIATED ENHANCEMENTS IN LIGHT SENSITIVITY IN ROD-CONE DYSTROPHY (RCD)**

(71) Applicant: Sorbonne Université, 75006 Paris (FR); Centre National de la Recherche Scientifique, 75016 Paris (FR); INSERM (Institut National de la Santé et de la Recherche Médicale), 75013 Paris (FR); Ruhr-Universität Bochum, 44801 Bochum (DE)
(72) Inventor: Dalkara, Deniz, 94140 Alfortville (FR); Sahel, José-Alain, 75013 Paris (FR); Herlitze, Stephan, 44797 Bochum (DE); Simon, Cardillia-Joe, 93270 Sevran (FR)
(74) Representative: Plasseraud IP

(57) **Abstract**

The present invention concerns a new gene therapy approach to increase light-sensitivity in degenerating cones in advanced stages of rod-cone dystrophy (RCD) mediated by G-protein-gated-K+ channel (GIRK), in particular GIRK2, activated by G proteins recruited by cone opsin expressed in degenerating cones.

SEQUENCE LISTING

<110>  Sorbonne Université
       Centre National de la Recherche Scientifique
       INSERM - Institut National de la Santé et de la Recherche
       Médicale
       Ruhr-Universität Bochum
       DALKARA, Deniz
       HERLITZE, Stephan
       SAHEL, José-Alain
       SIMON, Cardillia-Joe

<120>  G-PROTEIN-GATED-K+ CHANNEL-MEDIATED ENHANCEMENTS IN LIGHT
       SENSITIVITY IN ROD-CONE DYSTROPHY (RCD)

<130>  BNT228412EP00

<160>  6

<170>  PatentIn version 3.5

<210>  1
<211>  1269
<212>  DNA
<213>  Homo sapiens

<220>
<221>  CDS
<222>  (1)..(1269)

<400>  1
atg gcc aag ctg aca gaa tcc atg act aac gtc ctg gag ggc gac tcc      48
atg gat cag gac gtc gaa agc cca gtg gcc att cac cag cca aag ttg      96
cct aag cag gcc agg gat gac ctg cca aga cac atc agc cga gat cgg      144
acc aaa agg aaa atc cag agg tac gtg agg aaa gac gga aag tgc aat      192
gtt cat cac ggc aac gtg agg gag acc tat cgc tac ctg acc gat atc      240
ttc acc aca tta gtg gac ctg aag tgg aga ttc aac cta ttg att ttt      288
gtc atg gtt tac aca gtg acc tgg ctc ttt ttt gga atg atc tgg tgg      336
ttg atc gca tac ata cgg gga gac atg gac cac ata gag gac ccc tcc      384
tgg act cct tgt gtt acc aac ctc aac ggg ttc gtc tct gct ttt tta     432
ttc tca ata gag aca gaa acc acc att ggt tat ggc tac cgg gtc atc     480
aca gat aaa tgc cca gag gga att att ctt ctc tta atc caa tct gtg     528
ttg ggg tcc att gtc aat gca ttc atg gtg gga tgc atg ttt gta aaa     576
atc tct caa ccc aag aag agg gca gag acc ctg gtc ttt tcc acc cat     624
gca gtg atc tcc atg cgg gat ggg aaa ctg tgc ctg atg ttc cgg gta     672
ggg gac ctt agg aat tcc cac att gtg gag gct tcc atc aga gcc aag     720
ttg atc aaa tcc aaa cag acc tcg gag ggg gag ttc atc ccg ttg aac     768
cag acg gat atc aac gta ggg tat tac acg ggg gat gac cgt ctg ttt     816
ctg gtg tca ccg ctg atc att agc cat gaa att aac caa cag agt cct     864
ttc tgg gag atc tcc aaa gcc cag ctg ccc aaa gag gaa ctg gaa att     912
gtg gtc atc cta gaa gga atg gtg gaa gcc aca ggg atg aca tgc caa     960
gct cga agc tcc tac atc acc agt gag atc ctg tgg ggt tac cgg ttc     1008
aca cct gtc ctg acc ctg gag gac ggg ttc tac gaa gtt gac tac aac     1056
agc ttc cat gag acc tat gag acc agc acc cca tcc ctt agt gcc aaa     1104
gag ctg gcc gag tta gcc agc agg gca gag ctg ccc ctg agt tgg tct     1152
gta tcc agc aaa ctc aac caa cat gca gaa ctg gag act gaa gag gaa        1200
gaa aag aac ctc gaa gag caa aca gaa aga aat ggt gat gtg gca aac        1248
ctg gag aat gaa tcc aaa gtt                                            1269

<210>  2
<211>  423
<212>  PRT
<213>  Homo sapiens

<400>  2

<210>  3
<211>  1275
<212>  DNA
<213>  Mus musculus

<220>
<221>  CDS
<222>  (1)..(1275)

<400>  3
atg aca atg gcc aag tta act gaa tcc atg act aac gcc gcc gaa ggc        48
Met Thr Met Ala Lys Leu Thr Glu Ser Met Thr Asn Ala Ala Glu Gly
1               5                   10                  15

gat tcc atg gac cag gat gtg gaa agc cca gtg gcc att cac cag cca        96
Asp Ser Met Asp Gln Asp Val Glu Ser Pro Val Ala Ile His Gln Pro
            20                  25                  30

aag ttg cct aag cag gcc agg gac gac ctg ccg aga cac atc agc cga       144
Lys Leu Pro Lys Gln Ala Arg Asp Asp Leu Pro Arg His Ile Ser Arg
        35                  40                  45

gac agg acc aaa agg aaa atc cag agg tac gtg agg aag gat ggg aag       192
Asp Arg Thr Lys Arg Lys Ile Gln Arg Tyr Val Arg Lys Asp Gly Lys
    50                  55                  60

tgc aac gtt cac cac ggc aat gtg cgg gag acg tac cga tac ctg acg       240
Cys Asn Val His His Gly Asn Val Arg Glu Thr Tyr Arg Tyr Leu Thr
65                  70                  75                  80

gac atc ttc acc acc ctg gtg gac ctg aag tgg aga ttc aac ctg ttg       288
Asp Ile Phe Thr Thr Leu Val Asp Leu Lys Trp Arg Phe Asn Leu Leu
                85                  90                  95

atc ttt gtc atg gtc tac aca gtg acg tgg ctt ttc ttt ggg atg atc       336
Ile Phe Val Met Val Tyr Thr Val Thr Trp Leu Phe Phe Gly Met Ile
            100                 105                 110

tgg tgg ctg att gcg tac atc cgg gga gat atg gac cac ata gag gac       384
Trp Trp Leu Ile Ala Tyr Ile Arg Gly Asp Met Asp His Ile Glu Asp
        115                 120                 125

ccc tcg tgg act cct tgt gtc acc aac ctc aac ggg ttt gtc tct gct       432
Pro Ser Trp Thr Pro Cys Val Thr Asn Leu Asn Gly Phe Val Ser Ala
    130                 135                 140

ttt tta ttc tcc ata gag aca gaa acc acc atc ggt tat ggc tac cgg       480
Phe Leu Phe Ser Ile Glu Thr Glu Thr Thr Ile Gly Tyr Gly Tyr Arg
145                 150                 155                 160

gtc atc acg gac aag tgc cct gag ggg att att ctc ctc tta atc cag       528
Val Ile Thr Asp Lys Cys Pro Glu Gly Ile Ile Leu Leu Leu Ile Gln
                165                 170                 175

tcc gtg ttg ggg tcc att gtc aac gcc ttc atg gta gga tgt atg ttt       576
Ser Val Leu Gly Ser Ile Val Asn Ala Phe Met Val Gly Cys Met Phe
            180                 185                 190

gtg aaa ata tcc caa ccc aag aag agg gca gag acc ctg gtc ttt tcc 624
Val Lys Ile Ser Gln Pro Lys Lys Arg Ala Glu Thr Leu Val Phe Ser
        195                 200                 205

acc cac gcg gtg atc tcc atg cgg gat ggg aaa ctg tgc ttg atg ttc 672
Thr His Ala Val Ile Ser Met Arg Asp Gly Lys Leu Cys Leu Met Phe
    210                 215                 220

cgg gtg ggg gac ttg agg aat tct cac att gtg gag gca tcc atc aga 720
Arg Val Gly Asp Leu Arg Asn Ser His Ile Val Glu Ala Ser Ile Arg
225                 230                 235                 240

gcc aag ttg atc aag tcc aaa cag act tca gag ggg gag ttt att ccc 768
Ala Lys Leu Ile Lys Ser Lys Gln Thr Ser Glu Gly Glu Phe Ile Pro
                245                 250                 255

ctc aac cag act gat atc aac gtg ggg tac tac aca ggg gac gac cgg 816
Leu Asn Gln Thr Asp Ile Asn Val Gly Tyr Tyr Thr Gly Asp Asp Arg
            260                 265                 270

ctc ttt ctg gtg tca cca ttg att att agc cat gaa att aac caa cag 864
Leu Phe Leu Val Ser Pro Leu Ile Ile Ser His Glu Ile Asn Gln Gln
        275                 280                 285

agt ccc ttc tgg gag atc tcc aaa gcg cag ctg cct aaa gag gaa ctg 912
Ser Pro Phe Trp Glu Ile Ser Lys Ala Gln Leu Pro Lys Glu Glu Leu
    290                 295                 300

gag att gtg gtc atc ctg gag gga atg gtg gaa gcc aca gga atg acg 960
Glu Ile Val Val Ile Leu Glu Gly Met Val Glu Ala Thr Gly Met Thr
305                 310                 315                 320

tgc caa gcc cga agc tcc tac atc acc agt gag atc ttg tgg ggt tac 1008
Cys Gln Ala Arg Ser Ser Tyr Ile Thr Ser Glu Ile Leu Trp Gly Tyr
                325                 330                 335

cgg ttc aca cct gtc cta acg ctg gaa gac ggg ttc tac gaa gtt gac 1056
Arg Phe Thr Pro Val Leu Thr Leu Glu Asp Gly Phe Tyr Glu Val Asp
            340                 345                 350

tac aac agc ttc cat gag acc tat gag acc agc acc ccg tcc ctt agt 1104
Tyr Asn Ser Phe His Glu Thr Tyr Glu Thr Ser Thr Pro Ser Leu Ser
        355                 360                 365

gcc aaa gag cta gcg gag ctg gct aac cgg gca gag ctg cct ctg agt 1152
Ala Lys Glu Leu Ala Glu Leu Ala Asn Arg Ala Glu Leu Pro Leu Ser
    370                 375                 380

tgg tct gtg tcc agc aaa ctg aac caa cat gca gaa ttg gag aca gaa 1200
Trp Ser Val Ser Ser Lys Leu Asn Gln His Ala Glu Leu Glu Thr Glu
385                 390                 395                 400

gag gaa gag aag aac ccg gaa gaa ctg acg gag agg aat ggt gac gtg 1248
Glu Glu Glu Lys Asn Pro Glu Glu Leu Thr Glu Arg Asn Gly Asp Val
                405                 410                 415

gca aac cta gag aat gaa tcc aaa gtt 1275
Ala Asn Leu Glu Asn Glu Ser Lys Val
            420                 425

<210> 4
<211> 425

<212>  PRT
<213>  Mus musculus

<400>  4

Met Thr Met Ala Lys Leu Thr Glu Ser Met Thr Asn Ala Ala Glu Gly
1               5                   10                  15

Asp Ser Met Asp Gln Asp Val Glu Ser Pro Val Ala Ile His Gln Pro
            20                  25                  30

Lys Leu Pro Lys Gln Ala Arg Asp Asp Leu Pro Arg His Ile Ser Arg
        35                  40                  45

Asp Arg Thr Lys Arg Lys Ile Gln Arg Tyr Val Arg Lys Asp Gly Lys
    50                  55                  60

Cys Asn Val His His Gly Asn Val Arg Glu Thr Tyr Arg Tyr Leu Thr
65                  70                  75                  80

Asp Ile Phe Thr Thr Leu Val Asp Leu Lys Trp Arg Phe Asn Leu Leu
                85                  90                  95

Ile Phe Val Met Val Tyr Thr Val Thr Trp Leu Phe Phe Gly Met Ile
            100                 105                 110

Trp Trp Leu Ile Ala Tyr Ile Arg Gly Asp Met Asp His Ile Glu Asp
        115                 120                 125

Pro Ser Trp Thr Pro Cys Val Thr Asn Leu Asn Gly Phe Val Ser Ala
    130                 135                 140

Phe Leu Phe Ser Ile Glu Thr Glu Thr Thr Ile Gly Tyr Gly Tyr Arg
145                 150                 155                 160

Val Ile Thr Asp Lys Cys Pro Glu Gly Ile Ile Leu Leu Leu Ile Gln
                165                 170                 175

Ser Val Leu Gly Ser Ile Val Asn Ala Phe Met Val Gly Cys Met Phe
            180                 185                 190

Val Lys Ile Ser Gln Pro Lys Lys Arg Ala Glu Thr Leu Val Phe Ser
        195                 200                 205

Thr His Ala Val Ile Ser Met Arg Asp Gly Lys Leu Cys Leu Met Phe
    210                 215                 220

Arg Val Gly Asp Leu Arg Asn Ser His Ile Val Glu Ala Ser Ile Arg
225                 230                 235                 240

Ala Lys Leu Ile Lys Ser Lys Gln Thr Ser Glu Gly Glu Phe Ile Pro
                245                 250                 255

Leu Asn Gln Thr Asp Ile Asn Val Gly Tyr Tyr Thr Gly Asp Asp Arg
            260                 265                 270

Leu Phe Leu Val Ser Pro Leu Ile Ile Ser His Glu Ile Asn Gln Gln
        275                 280                 285

Ser Pro Phe Trp Glu Ile Ser Lys Ala Gln Leu Pro Lys Glu Glu Leu
    290                 295                 300

Glu Ile Val Val Ile Leu Glu Gly Met Val Glu Ala Thr Gly Met Thr
305                 310                 315                 320

Cys Gln Ala Arg Ser Ser Tyr Ile Thr Ser Glu Ile Leu Trp Gly Tyr
                325                 330                 335

Arg Phe Thr Pro Val Leu Thr Leu Glu Asp Gly Phe Tyr Glu Val Asp
            340                 345                 350

Tyr Asn Ser Phe His Glu Thr Tyr Glu Thr Ser Thr Pro Ser Leu Ser
        355                 360                 365

Ala Lys Glu Leu Ala Glu Leu Ala Asn Arg Ala Glu Leu Pro Leu Ser
    370                 375                 380

Trp Ser Val Ser Ser Lys Leu Asn Gln His Ala Glu Leu Glu Thr Glu
385                 390                 395                 400

Glu Glu Glu Lys Asn Pro Glu Glu Leu Thr Glu Arg Asn Gly Asp Val
                405                 410                 415

Ala Asn Leu Glu Asn Glu Ser Lys Val
            420                 425

<210>  5
<211>  1221
<212>  DNA
<213>  Rattus rattus

<220>
<221>  CDS
<222>  (1)..(1221)

<400>  5
atg gac caa gac gtg gaa agc cca gtg gcc att cac cag cca aag ttg        48
Met Asp Gln Asp Val Glu Ser Pro Val Ala Ile His Gln Pro Lys Leu

1                   5                   10                  15

cct aag cag gcc agg gat gac ctg cca aga cac atc agc cga gac agg      96
Pro Lys Gln Ala Arg Asp Asp Leu Pro Arg His Ile Ser Arg Asp Arg
            20                  25                  30

acc aaa agg aga atc cag agg tac gtg agg aag gat ggg aag tgt aac      144
Thr Lys Arg Arg Ile Gln Arg Tyr Val Arg Lys Asp Gly Lys Cys Asn
        35                  40                  45

gtc cac cac ggc aac gtg cgg gag acg tac cga tac ctg acg gac atc      192
Val His His Gly Asn Val Arg Glu Thr Tyr Arg Tyr Leu Thr Asp Ile
    50                  55                  60

ttc acc acc ctg gtg gac cta aag tgg aga ttc aac cta ttg atc ttt      240
Phe Thr Thr Leu Val Asp Leu Lys Trp Arg Phe Asn Leu Leu Ile Phe
65                  70                  75                  80

gtc atg gtc tac aca gtg acg tgg ctt ttc ttt ggg atg atc tgg tgg      288
Val Met Val Tyr Thr Val Thr Trp Leu Phe Phe Gly Met Ile Trp Trp
                85                  90                  95

cta att gca tac atc cgg gga gat atg gac cac ata gag gac ccc tcg      336
Leu Ile Ala Tyr Ile Arg Gly Asp Met Asp His Ile Glu Asp Pro Ser
            100                 105                 110

tgg act ccc tgt gtt acc aac ctc aac ggg ttt gtc tcc gct ttt tta      384
Trp Thr Pro Cys Val Thr Asn Leu Asn Gly Phe Val Ser Ala Phe Leu
        115                 120                 125

ttc tca ata gag aca gaa acc acc att ggt tat ggc tac agg gtc atc      432
Phe Ser Ile Glu Thr Glu Thr Thr Ile Gly Tyr Gly Tyr Arg Val Ile
    130                 135                 140

acg gac aag tgc cca gaa gga att att ctc ctc tta atc cag tcc gtg      480
Thr Asp Lys Cys Pro Glu Gly Ile Ile Leu Leu Leu Ile Gln Ser Val
145                 150                 155                 160

ttg ggg tcc att gtc aac gcc ttc atg gta gga tgt atg ttt gtg aaa      528
Leu Gly Ser Ile Val Asn Ala Phe Met Val Gly Cys Met Phe Val Lys
                165                 170                 175

ata tcc caa ccc aag aag agg gca gag acc ctg gtc ttt tcc acc cat      576
Ile Ser Gln Pro Lys Lys Arg Ala Glu Thr Leu Val Phe Ser Thr His
            180                 185                 190

gcg gta atc tcc atg cgg gat ggg aaa cta tgc ctg atg ttc cgg gta      624
Ala Val Ile Ser Met Arg Asp Gly Lys Leu Cys Leu Met Phe Arg Val
        195                 200                 205

ggg gac ttg agg aat tcc cac att gtg gag gcc tcc atc aga gcc aag      672
Gly Asp Leu Arg Asn Ser His Ile Val Glu Ala Ser Ile Arg Ala Lys
    210                 215                 220

ttg atc aag tcc aaa cag act tca gag ggg gag ttc att ccc ctc aac      720
Leu Ile Lys Ser Lys Gln Thr Ser Glu Gly Glu Phe Ile Pro Leu Asn
225                 230                 235                 240

cag acg gat atc aac gta ggg tac tac acc ggg gat gac cga ctc ttt      768
Gln Thr Asp Ile Asn Val Gly Tyr Tyr Thr Gly Asp Asp Arg Leu Phe
                245                 250                 255

ctc gtg tca ccg ctg att att agc cat gaa att aac caa cag agt ccc      816

Leu Val Ser Pro Leu Ile Ile Ser His Glu Ile Asn Gln Gln Ser Pro
            260                 265                 270

ttc tgg gag atc tcc aaa gcc cag ctg cct aaa gag gaa ctg gag att      864
Phe Trp Glu Ile Ser Lys Ala Gln Leu Pro Lys Glu Glu Leu Glu Ile
        275                 280                 285

gtg gtc atc ctg gag gga atg gtg gaa gcc aca gga atg acg tgc caa      912
Val Val Ile Leu Glu Gly Met Val Glu Ala Thr Gly Met Thr Cys Gln
    290                 295                 300

gct cga agc tcc tac gtc acc agt gag atc ctg tgg ggt tac cgg ttc      960
Ala Arg Ser Ser Tyr Val Thr Ser Glu Ile Leu Trp Gly Tyr Arg Phe
305                 310                 315                 320

aca cca gtc ctg aca ctg gag gac ggg ttc tat gaa gtt gac tac aac      1008
Thr Pro Val Leu Thr Leu Glu Asp Gly Phe Tyr Glu Val Asp Tyr Asn
                325                 330                 335

agc ttc cat gag acc cat gag acc agc acc ccg tcc ctt agc gcc aaa      1056
Ser Phe His Glu Thr His Glu Thr Ser Thr Pro Ser Leu Ser Ala Lys
            340                 345                 350

gag cta gcc gag ctg gct aac cgg gca gag ctg ccc ctg agc tgg tct      1104
Glu Leu Ala Glu Leu Ala Asn Arg Ala Glu Leu Pro Leu Ser Trp Ser
        355                 360                 365

gtg tcc agc aaa ctg aac caa cat gca gaa ctg gag acg gaa gag gaa      1152
Val Ser Ser Lys Leu Asn Gln His Ala Glu Leu Glu Thr Glu Glu Glu
    370                 375                 380

gag aag aac ccg gaa gaa ctg aca gag agg aat ggt gat gtg gca aac      1200
Glu Lys Asn Pro Glu Glu Leu Thr Glu Arg Asn Gly Asp Val Ala Asn
385                 390                 395                 400

cta gag aat gag tcc aaa gtg                                          1221
Leu Glu Asn Glu Ser Lys Val
                405

<210>  6
<211>  407
<212>  PRT
<213>  Rattus rattus

<400>  6

Met Asp Gln Asp Val Glu Ser Pro Val Ala Ile His Gln Pro Lys Leu
1               5                   10                  15

Pro Lys Gln Ala Arg Asp Asp Leu Pro Arg His Ile Ser Arg Asp Arg
            20                  25                  30

Thr Lys Arg Arg Ile Gln Arg Tyr Val Arg Lys Asp Gly Lys Cys Asn
        35                  40                  45

Val His His Gly Asn Val Arg Glu Thr Tyr Arg Tyr Leu Thr Asp Ile
    50                  55                  60

Phe Thr Thr Leu Val Asp Leu Lys Trp Arg Phe Asn Leu Leu Ile Phe
65                  70                  75                  80

Val Met Val Tyr Thr Val Thr Trp Leu Phe Phe Gly Met Ile Trp Trp
                85                  90                  95

Leu Ile Ala Tyr Ile Arg Gly Asp Met Asp His Ile Glu Asp Pro Ser
            100                 105                 110

Trp Thr Pro Cys Val Thr Asn Leu Asn Gly Phe Val Ser Ala Phe Leu
        115                 120                 125

Phe Ser Ile Glu Thr Glu Thr Thr Ile Gly Tyr Gly Tyr Arg Val Ile
    130                 135                 140

Thr Asp Lys Cys Pro Glu Gly Ile Ile Leu Leu Leu Ile Gln Ser Val
145                 150                 155                 160

Leu Gly Ser Ile Val Asn Ala Phe Met Val Gly Cys Met Phe Val Lys
                165                 170                 175

Ile Ser Gln Pro Lys Lys Arg Ala Glu Thr Leu Val Phe Ser Thr His
            180                 185                 190

Ala Val Ile Ser Met Arg Asp Gly Lys Leu Cys Leu Met Phe Arg Val
        195                 200                 205

Gly Asp Leu Arg Asn Ser His Ile Val Glu Ala Ser Ile Arg Ala Lys
    210                 215                 220

Leu Ile Lys Ser Lys Gln Thr Ser Glu Gly Glu Phe Ile Pro Leu Asn
225                 230                 235                 240

Gln Thr Asp Ile Asn Val Gly Tyr Tyr Thr Gly Asp Asp Arg Leu Phe
                245                 250                 255

Leu Val Ser Pro Leu Ile Ile Ser His Glu Ile Asn Gln Gln Ser Pro
            260                 265                 270

Phe Trp Glu Ile Ser Lys Ala Gln Leu Pro Lys Glu Glu Leu Glu Ile
        275                 280                 285

Val Val Ile Leu Glu Gly Met Val Glu Ala Thr Gly Met Thr Cys Gln
    290                 295                 300

Ala Arg Ser Ser Tyr Val Thr Ser Glu Ile Leu Trp Gly Tyr Arg Phe
305                 310                 315                 320

Thr Pro Val Leu Thr Leu Glu Asp Gly Phe Tyr Glu Val Asp Tyr Asn
                325                 330                 335

Ser Phe His Glu Thr His Glu Thr Ser Thr Pro Ser Leu Ser Ala Lys
            340                 345                 350

Glu Leu Ala Glu Leu Ala Asn Arg Ala Glu Leu Pro Leu Ser Trp Ser
        355                 360                 365

Val Ser Ser Lys Leu Asn Gln His Ala Glu Leu Glu Thr Glu Glu Glu
    370                 375                 380

Glu Lys Asn Pro Glu Glu Leu Thr Glu Arg Asn Gly Asp Val Ala Asn
385                 390                 395                 400

Leu Glu Asn Glu Ser Lys Val
                405

## Description

### Technical field of the invention

The present invention concerns a new gene therapy approach to increase light-sensitivity in degenerating cones in advanced stages of rod-cone dystrophy (RCD) mediated by G-protein-gated-K+ channel (GIRK), in particular GIRK2, activated by G proteins recruited by cone opsin expressed in degenerating cones.

In the description below, references in square brackets ([]) refer to the list of references at the end of the text.

### State of the art

Retina is the light sensitive tissue of the eye composed of three layers of neurons interconnected by synapses. The primary neurons of the retina are the light-sensing photoreceptors (PR), which are of two types: the rods for night vision and the cones for daylight vision. Cone-mediated vision is mostly supported by the fovea and is responsible for high acuity central vision most valuable to our daily visual tasks (Sinha et al., 2017) [1]. The light sensitive G protein coupled receptors that link photon capture to intracellular signaling leading to membrane hyperpolarization in photoreceptors are called opsins (Yau and Hardie, 2009) [2]. There is one type of rod opsin found in rods and three types of cone opsins - responsible for trichromatic vision - in the primate retina. The structural properties and phototransduction cascades are similar between these opsins.

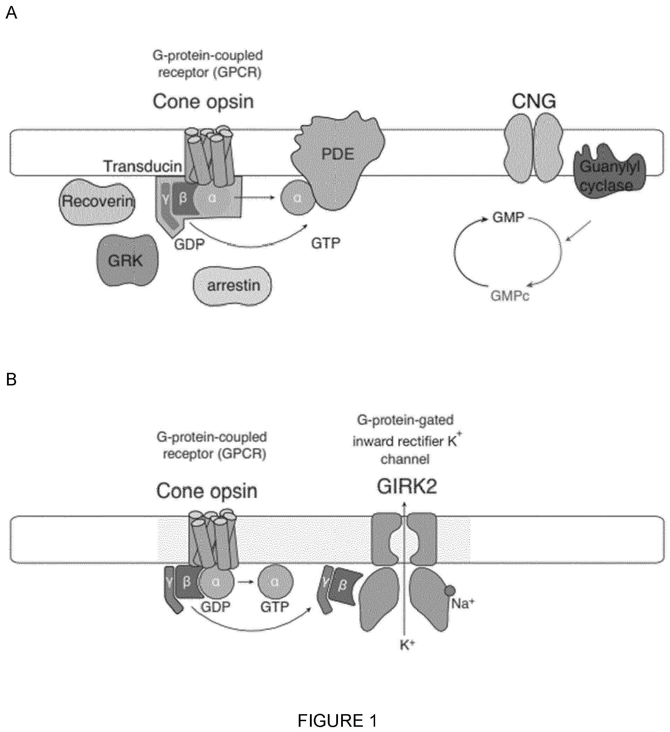

The phototransduction cascade is composed of several proteins that are concentrated in the photoreceptor outer-segments in normal retinas (Figure 1A). The role of the photoreceptor is to sense light via this phototransduction cascade and induce an electrical signal that is then processed and transmitted towards downstream neurons (Ebrey and Koutalos, 2001) [3].

The absorption of a photon activates the opsin composed of two parts: the protein part, and the light absorbing part, which is the retinal - a derivative of vitamin A. The latter isomerizes from 11-cis-retinal (dark adapted state) into all-trans-retinal configuration (light adapted state). As a result, the opsin becomes catalytically active recruiting the G protein transducin. The α-subunit of transducin is activated by the replacement of GDP by GTP. After, the α-subunit dissociates from the By-subunits to activate the membrane-associated phosphodiesterase 6 (PDE) by binding its two inhibitory γ subunits. The activated PDE hydrolyses cGMP into GMP. The reduction of cGMP clones the nucleotide-gated channels (CNG) and this stops cation entry, resulting in PR hyperpolarization and reduction in glutamate release by the photoreceptor (Larhammar et al., 2009) [4].

In order to respond to another photon, this phototransduction cascade is deactivated by two mechanisms: (i) the transducin inactivates itself by hydrolyzing the bound GTP and (ii) the rhodopsin kinase (GRK) phosphorylates the opsin that interacts with the regulatory protein arrestin, leading to opsin inactivation. Retinal is then recycled by the retinal pigment epithelium (RPE) and Müller glial cells. Each and every protein of this cascade plays an important role in converting the light signal into an electrical signal conveyed to the second and third order neurons (Maeda et al., 2003) [5].

Inherited retinal degenerations are mostly due to mutations in photoreceptor or RPE cells leading to the degeneration of the rods, followed by cone outer segment degeneration eventually leading to blindness (Buch et al., 2004) [6]. Among them, rod-cone dystrophy (RCD) represents the largest category where genetic causes are highly heterogeneous. More than 60 different genes expressed in rod photoreceptors or the retinal pigment epithelium are involved (Wright et al., 2010) [7]. The first gene that has been linked to RCD is the rhodopsin gene RHO that counts for 25% of RCD autosomal dominant cases. Many other causative genes have also been identified: the cGMP-PDE subunit gene and the cyclic GMP gated channel protein α subunit gene. Although there are many causative genes, the resulting RCD phenotype is the same across different mutations (Ferrari et al., 2011) [8]. The common RCD phenotype is characterized by the progressive rod degeneration, causing night blindness, and followed by progressive peripheral cone degeneration, causing "tunnel vision", mediated entirely by the remaining foveal cones then eventually resulting in complete blindness in the latest stages of disease. Usually, when patients are diagnosed with RCD, they already show night blindness, meaning their rods have degenerated. However, the cone remain until the late stages of the disease; particularly in the foveal region responsible for high acuity leading to tunnel vision in early stages (Li et al., 1995) [9]. In later stages of the disease, these cones lose their outer segment structures leading to complete blindness before the complete loss of the cone soma and pedicle (Li et al., 1995) [9].

In order to preserve the vision in these patients presenting light sensitive cone cell bodies, one innovative strategy is retinal gene therapy, which broadly refers to the transfer of a therapeutic gene into retinal cells to mediate a therapeutic effect (Bennet, 2017) [10]. Although the first successful clinical trials of gene therapy have focused on gene replacement, where a gene carrying a recessive mutation is replaced by a functional cDNA copy, this strategy is limited because it cannot be used for the majority of retinal degenerations (Bennet, 2017) [10]. For example, in RDC, the huge variability of mutations makes it difficult to apply to each specific mutation. Moreover, dominant mutations cannot be treated using this approach. Furthermore, in 50% of cases, the causative mutation is not elucidated or the rod photoreceptors bearing the most frequent mutations are already lost (Dalkara et al., 2015) [11]. For these reasons, mutation-independent gene therapies that can be applied beyond the loss of rods must be developed to treat a large number of patients without knowledge of the mutant gene.

Taking this goal into account, previous studies used the ectopic expression of microbial opsins, like channelrhodopsin in bipolar cells or halorhodopsin in cones PRs, to modulate membrane potential and induce a depolarization or hyperpolarization respectively (Busskamp et al., 2012; Dalkara and Sahel, 2014; Scholl et al., 2016) [12-14]. Thus, in RCD, optogenetics can be used as a therapeutic strategy to restore vision in blind retinas (Baker and Flannery, 2008) [15]. There are many parameters to consider: (i) the choice of cell target to make the most out of the retinal circuitry sending the most interpretable signal to the brain (ii) the choice of the appropriate optogenetic tool to obtain a close to natural electrophysiological response in these target cells. Concerning the second point, the relatively weak capacity of microbial opsins has been a major limitation: potential immunogenicity of using an opsin from prokaryotic species, high intensities required due to the lack of signal amplification by these directly light gated channels and pumps originating from single cell microorganisms, as in the case of halorhodopsin (Baker and Flannery, 2008) [15], (Cehajic-Kapetanovic et al., 2015; Gaub et al., 2015; Van Gelder and Kaur, 2015; van Wyk et al., 2015) [16-19]. Unlike rhodopsins or cone opsins, microbial opsins are not able to activate G protein coupled cascades such as the phototransduction cascade present in healthy retinas. One of the possible ways to go beyond the limits of microbial opsins is the use of animal opsins, which are all G protein coupled receptors. However all work in this field has so far been focused on inner retinal neurons (Berry et al., 2019; Cehajic-Kapetanovic et al., 2015; De Silva et al., 2017; Gaub et al., 2015; Lin et al., 2008; van Wyk et al., 2015) [20, 16, 21, 17, 22, 19].

In a previous study, it has been shown that light-activation of two animal cone opsins can stimulate the G_{i/o} signalling pathways in human kidney cells and in neuronal cells *in vitro* and *in vivo* (Masseck et al., 2014) [23]. This pathway is involved in fast dampening of neuronal activity and inhibition of intrinsic ion channels. However, it has also been shown that animal cone opsins can directly modulate the G protein-gated inwardly rectifying potassium channels (GIRK) upon co-expression in any given cell (Berry et al., 2019; Masseck et al., 2014) [20, 23]. GIRK channels are composed of two subunits. There are four types of subunits: GIRK1 to 4. GIRK1 and 3 cannot form homotetramers; they have to be associated with GIRK2 to be functional (Mark and Herlitze, 2000) [24]. Conversely, GIRK2 alone can form homotetramers. The GIRK channel is predominantly closed at resting membrane potentials. After its activation by the βγ subunit of a G_{i/o} protein, potassium ions flow out of the cell, thus, hyperpolarizing the neuron (Figure 1B). It has therefore been possible to use vertebrate cone opsins SWO (for short wavelength opsin) and LWO (for long wavelength opsin) for repetitive G_{i/o} activation of a specific wavelength *in vivo* in the anxiety circuitry, and the combination of cone opsins with GIRK has proven more efficient that microbial opsins at low intensities (Masseck et al., 2014) [23].

### Description of the invention

The Inventors have thus investigated if it was possible to implement this cone opsin based system in a vision restoration setting. Therefore in order to develop a light-sensitive cone reactivation strategy, the expression of the phototransduction cascade elements in cones during degeneration was first examined in two RCD mouse models. After exploring the phosphotransduction cascade in two RCD mouse models, a target molecule approach acting via G_{i/o} proteins recruited by the activation of remaining cone opsin was proposed for the first time. It has thereby created a new "short phototransduction cascade" that is independent from the expression of PDE and transducin.

First, the state of the endogeneous phototransduction cascade in degenerating cones through the progression of disease was investigated. In both mouse models, only opsin and arrestin were found to migrate to the cone cell bodies after outer segment degeneration. Thus it was hypothesized that cone reactivation based on cone opsin signaling may be feasible, which in turn will allow to recover high sensitivity vision. It was found that endogenous cone opsins were still expressed at the level of the cone cell body suggesting the possibility of linking their activity to GIRK channels, in particular GIRK2 channel, even in absence of transducin and phosphodiesterase (Figure 1B). In this configuration, the opening of GIRK2 channel will allow the exit of potassium ions due to the resting membrane potential of dormant cones (Busskamp, 2010) [25]. K⁺ efflux via the GIRK2 channel will hyperpolarize the cones in response to light as it was seen in the two mouse models of RCD.

Next, the target GIRK2 channel activated by G proteins recruited by cone opsin was expressed in degenerating cones. Moreover, since the remaining opsin in the cone cell bodies is still functional and sufficient to induce a light response in the degenerated cones, the insertion of GIRK2 in all cones leads to light responses following the spectral properties of each of the opsins preserving color vision. The results pointed towards enhanced light-sensitivity in cones of RCD retinas during and after degeneration of cone outer-segments. The results thus demonstrated that vertebrate light sensitive proteins combined with GIRK channel, in particular GIRK2 channel, activated by an endogenous G protein, could improve the vision function in the two mouse models, as demonstrated by electroretinography and behaviour. This is the first time that a light insensitive mammalian ion channel has been linked to intrinsic opsins providing new avenues in vision restoration that can be implemented to increase light sensitivity even before complete outer segment degeneration. Since this new system makes use of intrinsic opsins expressed in degenerating cones, it also enables for the first time, color vision restoration/maintenance. This new approach has thus the potential to maintain and/or restore, high acuity and color vision requiring only low light intensities in human patients.

A clear advantage of microbial opsins is their robustness and millisecond scale kinetics (Packer et al., 2013) [26]. For systems using other opsins, it should be considered that in order to respond to another light stimulus, the cascade has to be deactivated to recover light sensitivity. In absence of this, cones may stay hyperpolarized after GIRK2 channel activation limiting their ability to modulate synaptic transmission at a movie rate compatible with motion vision. In the present approach, depolarization of the cones was made possible thanks to the arrestin that is still maintained at very late stages of the disease in both RCD models - essential point which was not known in the art before the present invention. This was noticeable in the flicker ERG traces showing responses of the retina during repetitive light stimuli and also by the improved optokinetic reflex of treated mice.

Lastly, the fact that incorporation of GIRK2 enhances existing light responses in cones even prior to complete outer segment loss offers the possibility of implementing this gene therapy in mid stages of the disease. Retinal degeneration in mice is much faster than in humans, thus a few days of therapeutic efficiency in mice is equal to several years in humans. Nonetheless, even when endogenous light responses disappear, retinas expressing GIRK2 are still able to respond to light, generating response amplitudes that correspond to remaining cone numbers. This suggests that patients with remaining foveal cones can benefit from this treatment even if they have no detectable light perception at the beginning of treatment. Thus this approach can be use so long as cone cells remain. Indeed a decrease in the response of treated cones to light stimuli was recorded, which was consistent with decrease in cone numbers and the fact that we did not transduce all cones due to subretinal injection further limited the beneficial effect. AAV vectors showing better lateral spread can be used to increase transduced cone numbers beyond the bleb (Khabou et al., 2018; Demande internationale WO 2018134168) [27, 28]. In order to increase the therapeutic window, neurotrophic factors can be implemented alongside the approach of the present invention. Indeed, AAV-mediated secretion of neurotrophic factors such as the rod-derived cone viability factor (RdCVF) have been shown to delay cone cell death and may be combined with GIRK2 mediated sensitization (Byrne et al., 2015) [29].

An object of the present invention is therefore a vector comprising a nucleotide sequence encoding subunit 2 of G-protein-gated inwardly rectifying potassium channel (GIRK2) or a functional derivative thereof. The vector of the present invention can further comprise a nucleotide sequence encoding a mammal cone opsin. For example, the mammal cone opsin is a short wavelength cone opsin (SWO) from *mus musculus.*

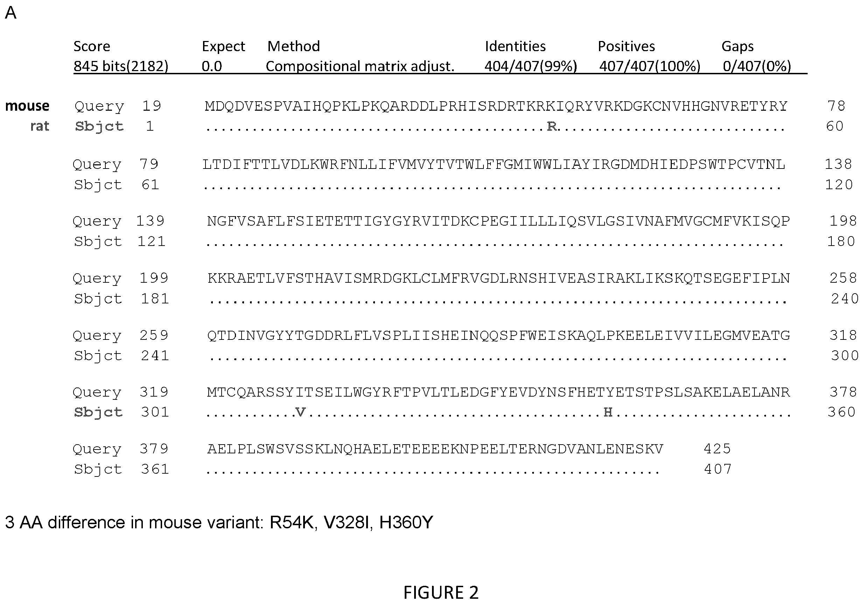

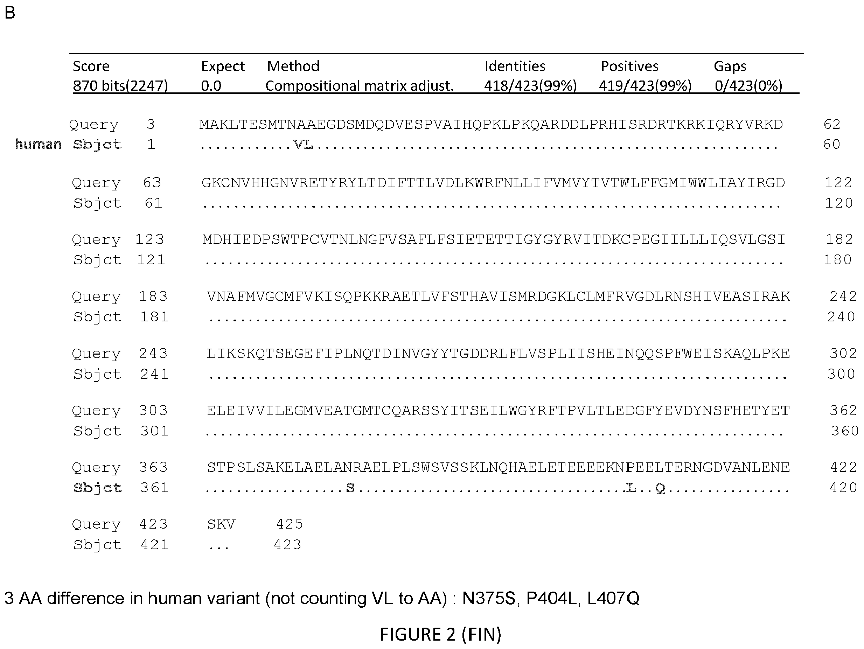

For the purposes of the present invention, "a functional derivative thereof" means a nucleotide sequence encoding an isoform or variant of GIRK2 which differs by only a few nucleotides compared to the WT form (e.g. mouse) or a nucleotide sequence encoding a truncated GIRK2 (Figure 2), but all of which retain the ability to respond to light when co-expressed with an opsin. For example, a nucleotide sequence encoding GIRK2 or a derivative thereof comprises or consists of the nucleotide sequence SEQ ID NOs: 1, 3 or 5.

| **Human GIRK2** | |
|---|---|
| Nucleotide sequence | ATGGCCAAGCTGACAGAATCCATGACTAACGTCCTGGAGGGCGACTCCATGGA TCAGGACGTCGAAAGCCCAGTGGCCATTCACCAGCCAAAGTTGCCTAAGCAGG CCAGGGATGACCTGCCAAGACACATCAGCCGAGATCGGACCAAAAGGAAAATC CAGAGGTACGTGAGGAAAGACGGAAAGTGCAATGTTCATCACGGCAACGTGAG GGAGACCTATCGCTACCTGACCGATATCTTCACCACATTAGTGGACCTGAAGTG GAGATTCAACCTATTGATTTTTGTCATGGTTTACACAGTGACCTGGCTCTTTTTG GAATGATCTGGTGGTTGATCGCATACATACGGGGAGACATGGACCACATAGAG GACCCCTCCTGGACTCCTTGTGTTACCAACCTCAACGGGTTCGTCTCTGCTTTTT TATTCTCAATAGAGACAGAAACCACCATTGGTTATGGCTACCGGGTCATCACAG ATAAATGCCCAGAGGGAATTATTCTTCTCTTAATCCAATCTGTGTTGGGGTCCAT TGTCAATGCATTCATGGTGGGATGCATGTTTGTAAAAATCTCTCAACCCAAGAAG AGGGCAGAGACCCTGGTCTTTTCCACCCATGCAGTGATCTCCATGCGGGATGG GAAACTGTGCCTGATGTTCCGGGTAGGGGACCTTAGGAATTCCCACATTGTGGA GGCTTCCATCAGAGCCAAGTTGATCAAATCCAAACAGACCTCGGAGGGGGAGTT CATCCCGTTGAACCAGACGGATATCAACGTAGGGTATTACACGGGGGATGACC GTCTGTTTCTGGTGTCACCGCTGATCATTAGCCATGAAATTAACCAACAGAGTCC TTTCTGGGAGATCTCCAAAGCCCAGCTGCCCAAAGAGGAACTGGAAATTGTGGT CATCCTAGAAGGAATGGTGGAAGCCACAGGGATGACATGCCAAGCTCGAAGCT CCTACATCACCAGTGAGATCCTGTGGGGTTACCGGTTCACACCTGTCCTGACCC TGGAGGACGGGTTCTACGAAGTTGACTACAACAGCTTCCATGAGACCTATGAGA CCAGCACCCCATCCCTTAGTGCCAAAGAGCTGGCCGAGTTAGCCAGCAGGGCA GAGCTGCCCCTGAGTTGGTCTGTATCCAGCAAACTCAACCAACATGCAGAACTG GAGACTGAAGAGGAAGAAAAGAACCTCGAAGAGCAAACAGAAAGAAATGGTGAT GTGGCAAACCTGGAGAATGAATCCAAAGTT (SEQ ID NO : 1) |
| Amino acid sequence (423Aa) |  (SEQ ID NO : 2) |

| **Mouse GIRK2** | |
|---|---|
| Nucleotide sequence | ATGACAATGGCCAAGTTAACTGAATCCATGACTAACGCCGCCGAAGGCGATTCC ATGGACCAGGATGTGGAAAGCCCAGTGGCCATTCACCAGCCAAAGTTGCCTAA GCAGGCCAGGGACGACCTGCCGAGACACATCAGCCGAGACAGGACCAAAAGG AAAATCCAGAGGTACGTGAGGAAGGATGGAAGTGCAACGTTCACCACGGCAA TGTGCGGAGACGTACCGATACCTGACGGACATCTTCACCACCCTGGTGGACC TGAAGTGGAGATTCAACCTGTTGATCTTTGTCATGGTCTACACAGTGACGTGGC |
| | TTTTCTTTGGGATGATCTGGTGGCTGATTGCGTACATCCGGGGAGATATGGACC ACATAGAGGACCCCTCGTGGACTCCTTGTGTCACCAACCTCAACGGGTTTGTCT CTGCTTTTTATTCTCCATAGAGACAGAAACCACCATCGGTTATGGCTACCGGGT CATCACGGACAAGTGCCCTGAGGGGATTATTCTCCTCTTAATCCAGTCCGTGTT GGGGTCCATTGTCAACGCCTTCATGGTAGGATGTATGTTTGTGAAAATATCCCAA CCCAAGAAGAGGGCAGAGACCCTGGTCTTTTCCACCCACGCGGTGATCTCCAT GCGGGATGGGAAACTGTGCTTGATGTTCCGGGTGGGGGACTTGAGGAATTCTC ACATTGTGGAGGCATCCATCAGAGCCAAGTTGATCAAGTCCAAACAGACTTCAG AGGGGGAGTTTATTCCCCTCAACCAGACTGATATCAACGTGGGGTACTACACAG GGACGACCGGCTCTTTCTGGTGTCACCATTGATTATTAGCCATGAAATTAACCA ACAGAGTCCCTTCTGGGAGATCTCCAAAGCGCAGCTGCCTAAAGAGGAACTGG AGATTGTGGTCATCCTGGAGGGAATGGTGGAAGCCACAGGAATGACGTGCCAA GCCCGAAGCTCCTACATCACCAGTGAGATCTTGTGGGGTTACCGGTTCACACCT GTCCTAACGCTGGAAGACGGGTTCTACGAAGTTGACTACAACAGCTTCCATGAG ACCTATGAGACCAGCACCCCGTCCCTTAGTGCCAAAGAGCTAGCGGAGCTGGC TAACCGGGCAGAGCTGCCTCTGAGTTGGTCTGTGTCCAGCAAACTGAACCAACA TGCAGAATTGGAGACAGAAGAGGAAGAGAAGAACCCGGAAGAACTGACGGAGA GGAATGGTGACGTGGCAAACCTAGAGAATGAATCCAAAGTT (SEQ ID NO : 3) |
| Amino acid sequence (425Aa) | **[two AA difference V¹³L¹⁴ to A¹³A¹⁴ compared to WT form (NCBI NP_034736.2)]** |
| | MTMAKLTESMTNAAEGDSMDQDVESPVAIHQPKLPKQARDDLPRHISRDRTKRKIQ RYVRKDGKCNVHHGNVRETYRYLTDIFTTLVDLKWRFNLLIFVMVYTVTWLFFGMIW WLIAYIRGDMDHIEDPSWTPCVTNLNGFVSAFLFSIETETTIGYGYRVITDKCPEGIILL LIQSVLGSIVNAFMVGCMFVKISQPKKRAETLVFSTHAVISMRDGKLCLMFRVGDLR NSHIVEASIRAKLIKSKQTSEGEFIPLNQTDINVGYYTGDDRLFLVSPLIISHEINQQSP FWEISKAQLPKEELEIVVILEGMVEATGMTCQARSSYITSEILWGYRFTPVLTLEDGF YEVDYNSFHETYETSTPSLSAKELAELANRAELPLSWSVSSKLNQHAELETEEEEKN PEELTERNGDVANLENESKV (SEQ ID NO : 4) |

| **Rat truncated GIRK2** | |
|---|---|
| Nucleotide sequence | ATGGACCAAGACGTGGAAAGCCCAGTGGCCATTCACCAGCCAAAGTTGCCTAA GCAGGCCAGGGATGACCTGCCAAGACACATCAGCCGAGACAGGACCAAAAGGA GAATCCAGAGGTACGTGAGGAAGGATGGGAAGTGTAACGTCCACCACGGCAAC GTGCGGGAGACGTACCGATACCTGACGGACATCTTCACCACCCTGGTGGACCT AAAGTGGAGATTCAACCTATTGATCTTTGTCATGGTCTACACAGTGACGTGGCTT TTCTTTGGGATGATCTGGTGGCTAATTGCATACATCCGGGGAGATATGGACCAC ATAGAGGACCCCTCGTGGACTCCCTGTGTTACCAACCTCAACGGGTTTGTCTCC GCTTTTTTATTCTCAATAGAGACAGAAACCACCATTGGTTATGGCTACAGGGTCA TCACGGACAAGTGCCCAGAAGGAATTATTCTCCTCTTAATCCAGTCCGTGTTGG GGTCCATTGTCAACGCCTTCATGGTAGGATGTATGTTTGTGAAAATATCCCAACC CAAGAAGAGGGCAGAGACCCTGGTCTTTTCCACCCATGCGGTAATCTCCATGCG GGATGGGAAACTATGCCTGATGTTCCGGGTAGGGGACTTGAGGAATTCCCACAT TGTGGAGGCCTCCATCAGAGCCAAGTTGATCAAGTCCAAACAGACTTCAGAGGG GGAGTTCATTCCCCTCAACCAGACGGATATCAACGTAGGGTACTACACCGGGGA TGACCGACTCTTTCTCGTGTCACCGCTGATTATTAGCCATGAAATTAACCAACAG AGTCCCTTCTGGGAGATCTCCAAAGCCCAGCTGCCTAAAGAGGAACTGGAGATT GTGGTCATCCTGGAGGGAATGGTGGAAGCCACAGGAATGACGTGCCAAGCTCG AAGCTCCTACGTCACCAGTGAGATCCTGTGGGGTTACCGGTTCACACCAGTCCT GACACTGGAGGACGGGTTCTATGAAGTTGACTACAACAGCTTCCATGAGACCCA TGAGACCAGCACCCCGTCCCTTAGCGCCAAAGAGCTAGCCGAGCTGGCTAACC GGGCAGAGCTGCCCCTGAGCTGGTCTGTGTCCAGCAAACTGAACCAACATGCA GAACTGGAGACGGAAGAGGAAGAGAAGAACCCGGAAGAACTGACAGAGAGGAA TGGTGATGTGGCAAACCTAGAGAATGAGTCCAAAGTG (SEQ ID NO : 5) |
| Amino acid sequence (407Aa) | **[lacks the first 18AA at N-terminal compared to WT form (NCBI P48550.1)]** |
| | MDQDVESPVAIHQPKLPKQARDDLPRHISRDRTKRRIQRYVRKDGKCNVHHGNVR ETYRYLTDIFTTLVDLKWRFNLLIFVMVYTVTWLFFGMIWWLIAYIRGDMDHIEDPSW TPCVTNLNGFVSAFLFSIETETTIGYGYRVITDKCPEGIILLLIQSVLGSIVNAFMVGCM FVKISQPKKRAETLVFSTHAVISMRDGKLCLMFRVGDLRNSHIVEASIRAKLIKSKQT SEGEFIPLNQTDINVGYYTGDDRLFLVSPLIISHEINQQSPFWEISKAQLPKEELEIVVI LEGMVEATGMTCQARSSYVTSEILWGYRFTPVLTLEDGFYEVDYNSFHETHETSTP SLSAKELAELANRAELPLSWSVSSKLNQHAELETEEEEKNPEELTERNGDVANLEN ESKV (SEQ ID NO : 6) |

Another object of the present invention is a carrier including a vector of the present invention.

According to a particular embodiment of the present invention, the carrier can include a vector comprising a nucleotide sequence encoding subunit 2 of G-protein-gated inwardly rectifying potassium channel (GIRK2) or a functional derivative thereof as described above and a vector comprising a nucleotide sequence encoding a mammal cone opsin. For example, the mammal cone opsin is a short wavelength cone opsin (SWO) from *mus musculus.*

According to a particular embodiment of the present invention, the carrier is for example chosen from solid-lipid nanoparticles, chitosan nanoparticles, liposome, lipoplex or cationic polymer.

According to a particular embodiment of the present invention, the vector of the present invention is a virus, chosen from an adeno-associated virus (AAV), an adenovirus, a lentivirus, an SV40 viral vector. According to a particular embodiment of the present invention, the present invention is equal to or less than 30 nm in size. For example it is an adeno-associated virus (AAV).

Another object of the present invention is a pharmaceutical composition comprising the vector or the carrier of the present invention, with a pharmaceutically acceptable carrier, diluent or excipient.

Another object of the present invention is a vector, a carrier or a pharmaceutical composition of the present invention, for use in treating rod-cone dystrophy (RCD). In other words, another object of the present invention is a method of treating a RCD in a mammal in need thereof, the method comprising administering to the mammal an effective amount of the vector or the carrier of the pharmaceutical composition of the present invention. Rod-cone dystrophy (RCD) is a heterogeneous group of diseases such as non-syndromic X-linked Retinitis Pigmentosa (XLRP), autosomal recessive RP, autosomal dominant RP. The most common syndromic forms of RCD include Usher syndrome, Refsum disease, Bassen-Kornzweig syndrome, Bardet-Biedl syndrome and Batten disease.

Another object of the present invention is a nucleotide sequence encoding subunit 2 of G-protein-gated inwardly rectifying potassium channel (GIRK2) or a derivative thereof as described above, for use as a medicament. In particular said nucleotide sequence is useful for treating rod-cone dystrophy (RCD). In other words, another object of the present invention is a method of treating a RCD in a mammal in need thereof, the method comprising administering to the mammal an effective amount of a nucleotide sequence encoding subunit 2 of G-protein-gated inwardly rectifying potassium channel (GIRK2) or a derivative thereof as described above.

### Brief description of the figures

Figure 1 represents phototransduction cascade (A) normal phototransduction cascade (B) short phototransduction cascade with an animal opsin and GIRK2 channel. PDE: phosphodiesterase. CNG: cyclic-nucleotic gated channels. cGMP: cyclic guanosine monophosphate.
Figure 2 represents alignments of GIRK2 (A) rat truncated GIRK2 vs mouse GIRK2 (B) mouse GIRK2 vs human GIRK2.

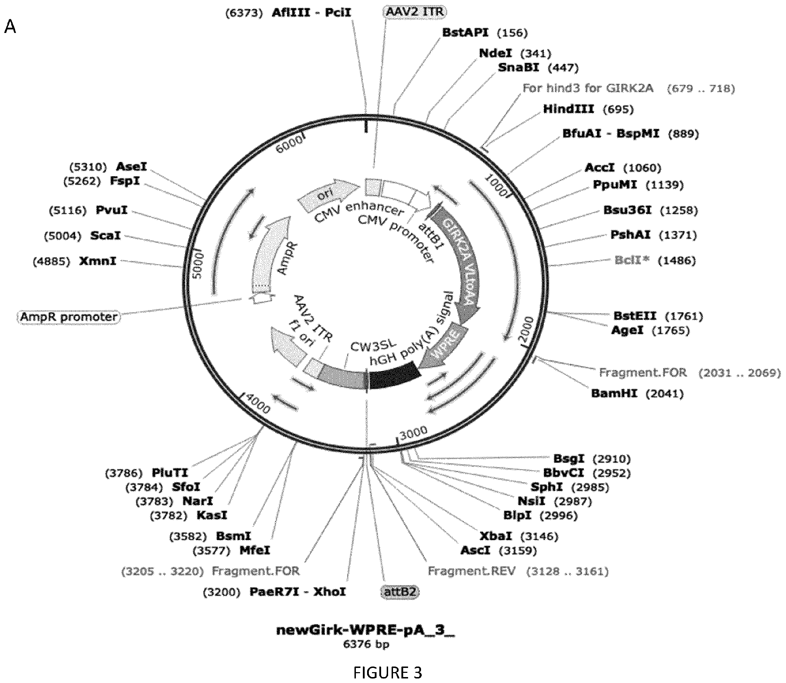

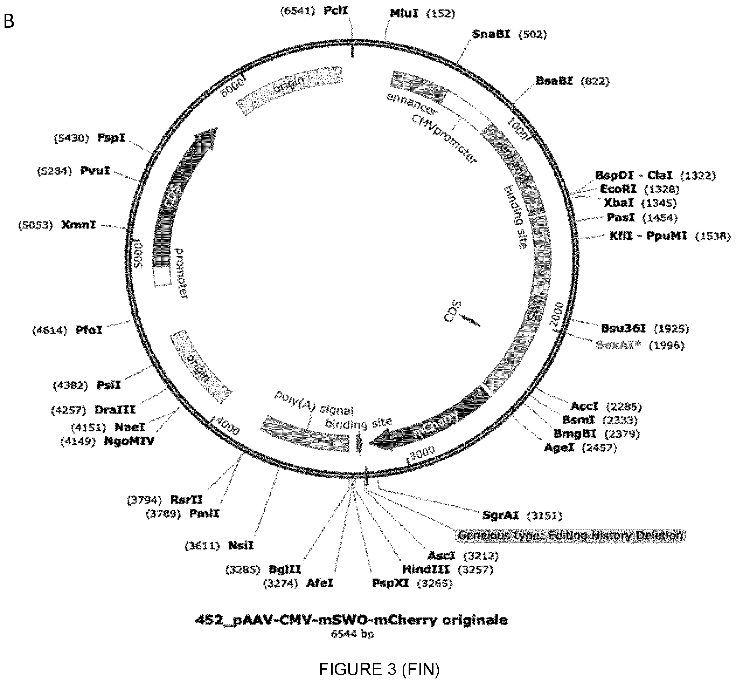

Figure 3 represents plasmids (A) CMV-GIRK2-GFP and (B) CMV-SWO-mCherry.

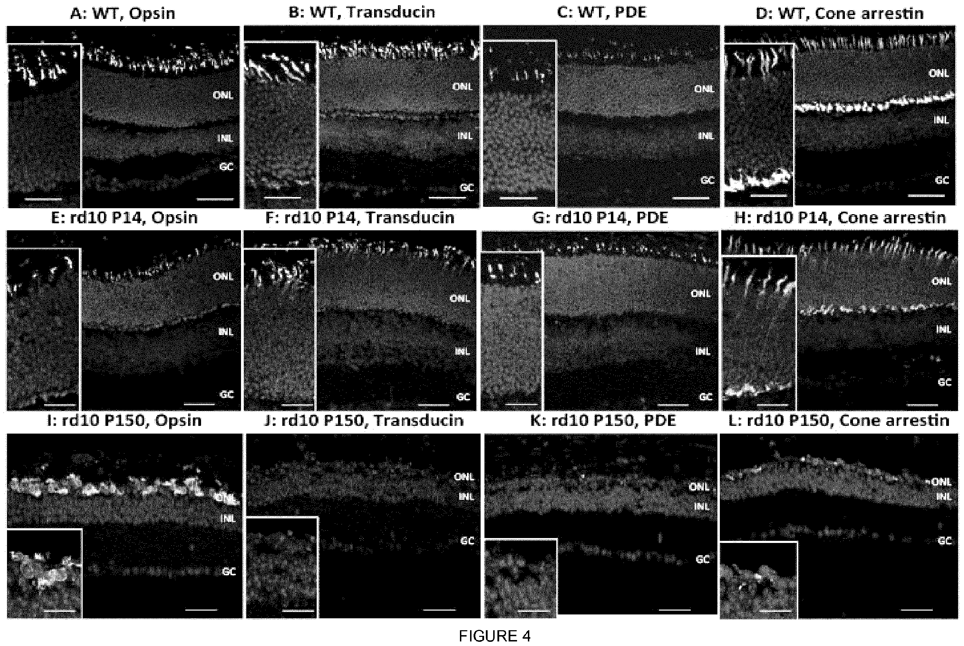

Figure 4 represents what remained in the phototransduction cascade in rd10 mice using immunohistochemistry (A-D) retinal cross-section of a control WT mouse stained with (A) opsin, (B) transducin, (C) PDE and (D) cone arrestin. (EH) retinal cross-section of a rd10 mouse at P14 stained with (E) opsin, (F) transducin, (G) PDE and (H) cone arrestin. (I-L) Retinal cross-section of a rd10 mouse at P150 stained with (I) opsin, (J) transducin, (K) PDE and (L) cone arrestin. ONL: outer nuclear layer. INL: inner nuclear layer. GC: ganglion cells. Scale bar is 50µm. Inset scale bar is 25µm.

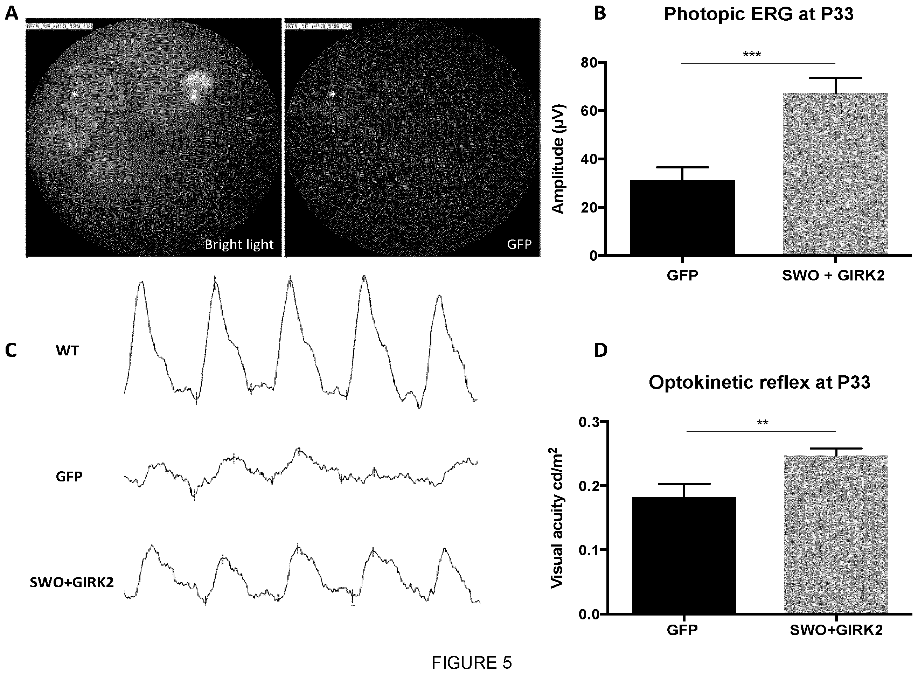

Figure 5 represents preliminary datas. (A) Eye fundus of GIRK2-GFP expression in rd 10 mouse one week post-injection *(***site of injection)* (B) Photopic ERG amplitude in rd10 mice at P33, injected with AAV-SWO-tdTomato and AAV-GIRK2-GFP. Control mice are injected with AAV-GFP (n=12). *P=0,0002.* (C) Representative flickers ERG at P33. (D) Measure of the visual acuity by optokinetic test in rd10 mice, injected with AAV-SWO-tdTomato and AAV-GIRK2-GFP Control mice are injected with AAV-GFP. Control mice were injected with AAV-GFP (n=8).

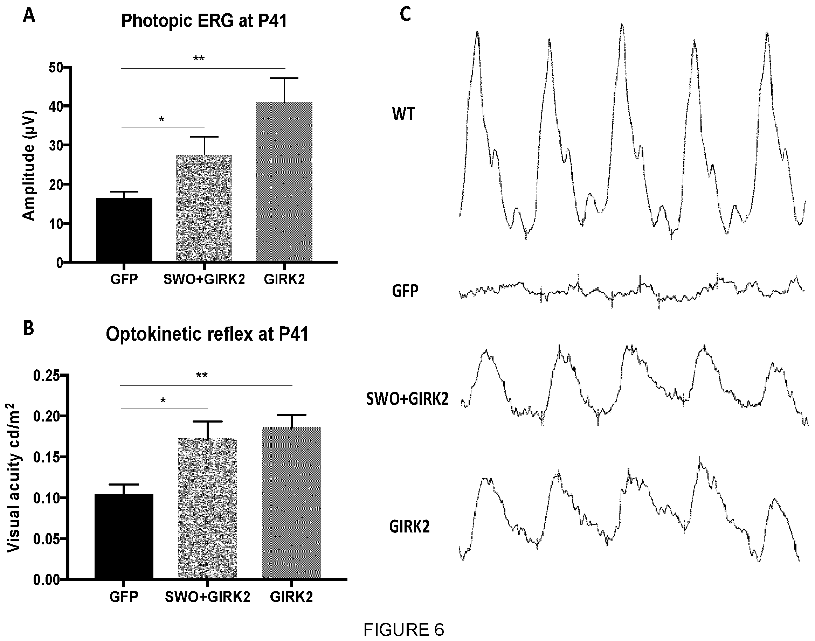

Figure 6 represents GIRK2-mediated vision. (A) Photopic ERG amplitude in rd 10 mice at P41, injected with AAV-SWO-tdTomato and/or AAV-GIRK2-GFP. Control mice are injected with AAV-GFP (n=12). *P*_{*SWO*+*GIRK2*} = *0,0381 and P_{GIRK2}*=*0,0021.* (B) Measure of the visual acuity by optokinetic test in rd10 mice, injected with AAV-SWO-tdTomato and/or AAV-GIRK2-GFP. Control mice are injected with AAV-GFP. Control mice were injected with AAV-GFP (n=7). (C) Representative flickers ERG at P41.

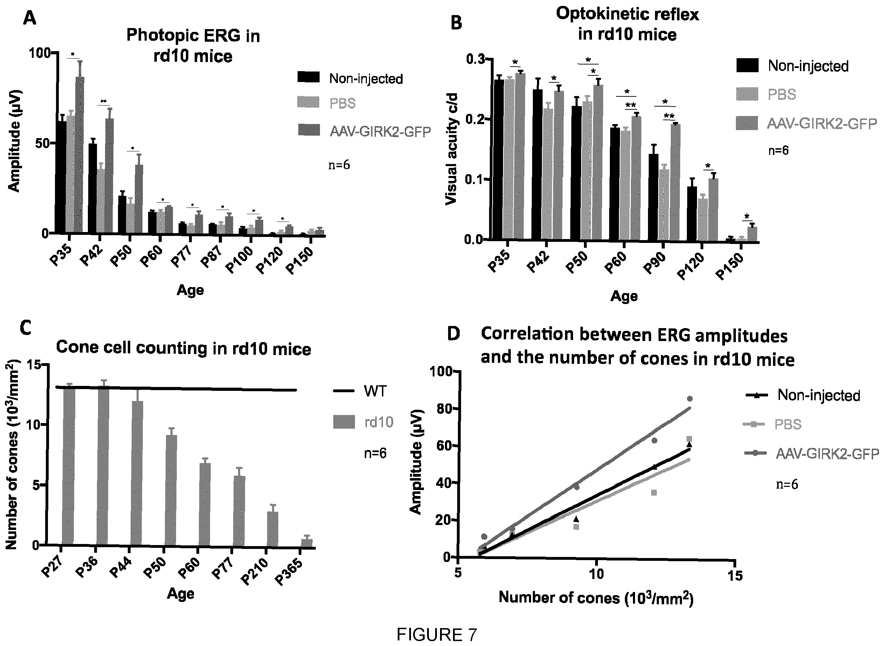

Figure 7 represents long term efficiency. (A) Photopic ERG amplitude in rd10 mice, injected with AAV-GIRK2-GFP Control mice are injected with PBS (n=6). (B) Measure of the visual acuity by optokinetic test in rd10 mice, injected with AAV-GIRK2-GFP. Control mice are injected with PBS (n=6). (C) Number of cones of wild-type mice and non-injected rd10 mice over time (n=6). *Pvalue*_{*(P50*-*P365*)} = *0.0022.* (D) Linear regression correlation between the ERG amplitudes and the number of cones in rd 10 mice (n=6). *Pvalue _{non-injected}* = *0,0482. Pvalue* _{*AAV*-*GIRK2*-*GFP*} = *0,0007. Pvalue _{PBS}* = *0,0104.*

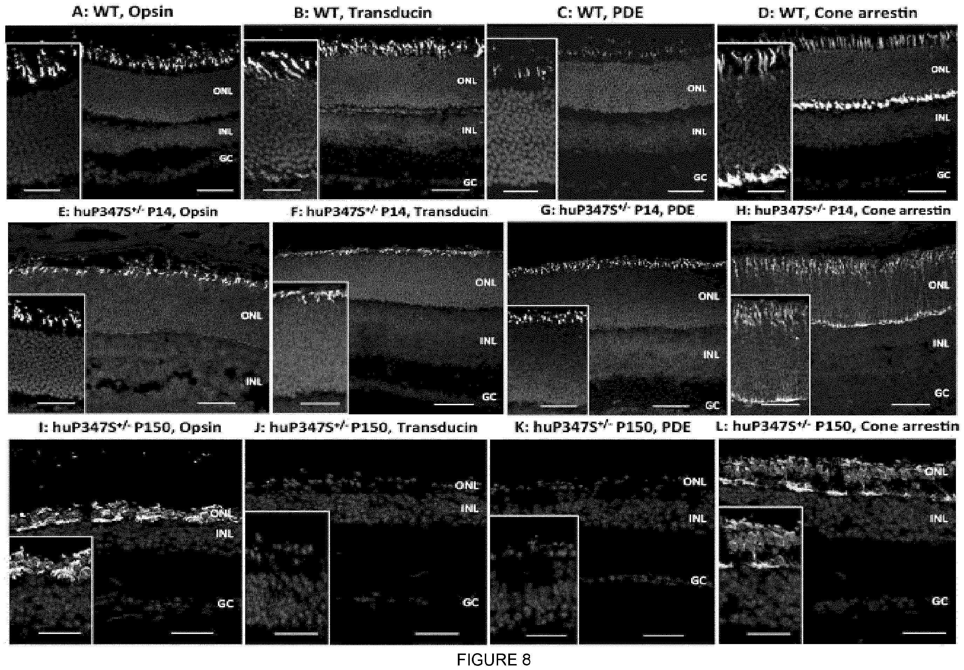

Figure 8 represents what remained in the phototransduction cascade in huP347S^{+/-} mice using immunohistochemistry. (A-D) Retinal cross-section of a control WT mouse stained with (A) opsin, (B) transducin, (C) PDE and (D) cone arrestin. (E-H) retinal cross-section of a huP347S^{+/-} mouse at P14 stained with (E) opsin, (F) transducin, (G) PDE and (H) cone arrestin. (I-L) retinal cross-section of a huP347S^{+/-} mouse at P150 stained with (I) opsin, (J) transducin, (K) PDE and (L) cone arrestin. ONL: outer nuclear layer. INL: inner nuclear layer. GC: ganglion cells. Scale bar is 50µm. Inset scale bar is 25µm.

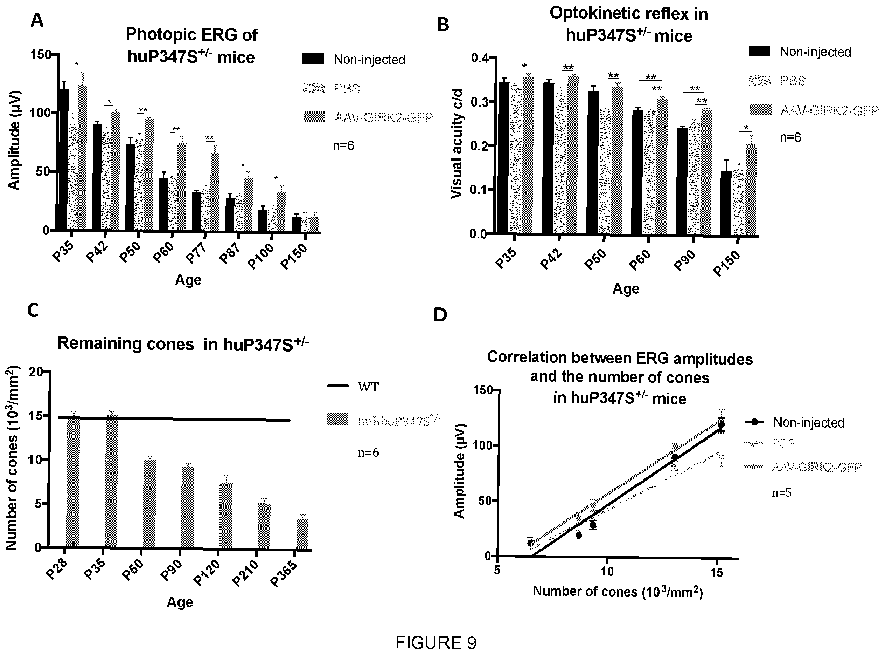

Figure 9 represents universality of the approach. (A) Photopic ERG amplitude in huP347S^{+/-} mice, injected with AAV-GIRK2-GFP. Control mice are injected with PBS (n=6). (B) Measure of the visual acuity by optokinetic test in huP347S^{+/-} mice, injected with AAV-GIRK2-GFP. Control mice are injected with PBS (n=6). (C) Number of cones of wild-type mice and non-injected huP347S^{+/-}mice over time (n=6). *Pvalue _{(P50-P365)}* = *0,0022.* (D) Linear regression correlation between the ERG amplitudes and the number of cones in huP347S^{+/-} mice (n=5). *Pvalue* _{*non*-}*_{injected} = 0,0313. Pvalue _{AAV-GIRK2-GFP} = 0,0146. Pvalue _{PBS} = 0,0497.*

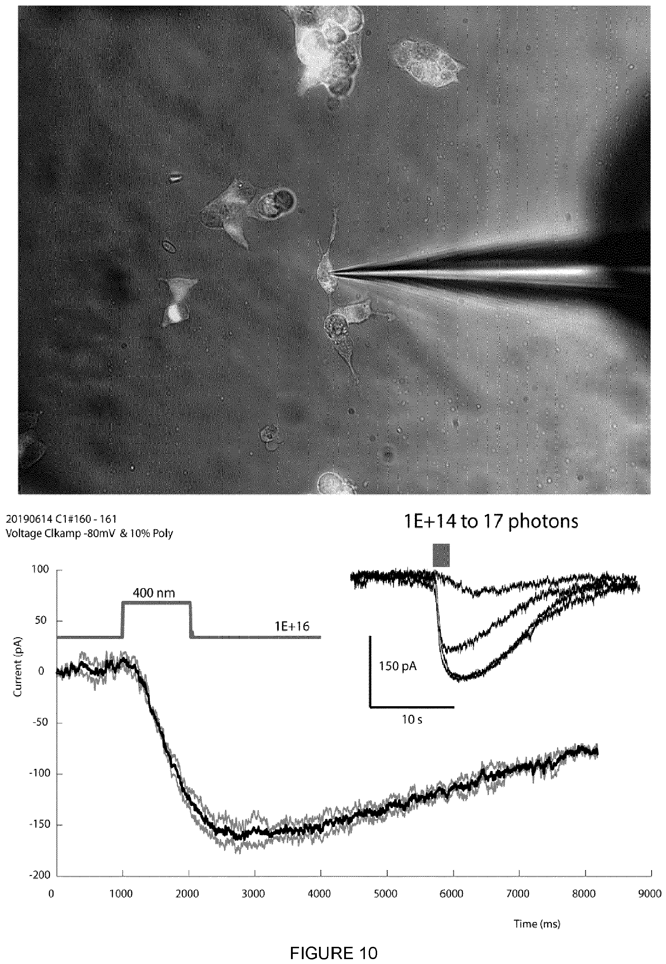

Figure 10 represents the efficiency of the mouse GIRK2 in HEK cells transfected with two plasmids: CMV-SWO-mCherry and CMV-GIRK2-GFP.

### EXAMPLES

### EXAMPLE 1 : MATERIAL AND METHODS

### 1. Animals

C57BL/6j^{rd10/rd10} (rd10) mice were used in these experiments. They have a mutation on the rod PDE gene leading to a dysfunctional phototransduction cascade and a rod-cone dystrophy. The second model used is the huRhoP347S^{+/-}mouse. The homozygous strand of this mouse present a KO of mouse rhodopsin (mRho) gene and a KI of human rhodopsin (huRho) with a mutation (P347S) (Millington-Ward et al., 2011) [30]. The homozygous males were crossed C57BL/6j (wild-type) females to obtain heterozygous mice. These mice have a similar phenotype as the rd 10 mice but the degeneration rate is lower.

### 2. AAV injections

Mice were first anesthetised with intraperitoneal injections of 0.2 ml/20g ketamine (Ketamine 500, Vibrac France) and xylazine (Xylazine 2%, Rompun) diluted in 0.9% NaCl. Eyes were dilated with 8% Neosynephrine (Neosynephrine Faure 10%, Europhta) and 42% Mydriaticum (Mydriaticum 0.5%, Thea) diluted in 0.9% NaCl.

A total volume of 1µl of vector solution was injected subretinally. Fradexam, an ophthalmic ointment, was applied after injection. The list of injected viral vectors is presented below:

| Injection Table | | | | |
|---|---|---|---|---|
| Mice | Eyes | viral vector injected | viral vector titration | volume injected |
| rd 10 | both | AAV8-mCAR-GFP | 10¹¹ rAAV particles | 1 µl for all conditions |
| rd10 | both | AAV8-mCAR-GIRK2-GFP+ AAV8-mCAR-SWO-tdTomato | | |
| rd10 | both | AAV8-mCAR-GIRK2-GFP | | |
| rd 10 | right | PBS | | |
| rd 10 | left | AAV8-PR1.7-GIRK2-GFP | 5.10⁸ rAAV particles | |
| huRhoP347S^{+/--} | right | PBS | | |
| huRhoP347S^{+/-} | left | AAV8-PR1.7-GIRK2-GFP | 5.10⁸ rAAV particles | |

### 3. Eye fundus examination

One week after subretinal injection, mice were anesthetised by isofluorane inhalation. Eyes were dilated and then protected with Lubrithal eye gel (VetXX). Fundus imaging was performed with a fundus camera (Micron III; Phoenix research Lab) equipped with specific filters to monitor GFP or tdTomato expression in live anesthetised mice.

### 4. Electroretinography (ERG) recordings

To evaluate retinal function, electroretinography recordings (ERG) were recorded (espion E2 ERG system; Diagnosys). Several tests were performed at different time points after injections of the viral vectors. Mice were anesthetised with intraperitoneal injections of 0.2 ml/20g ketamine (Ketamine 500, Vibrac France) and xylazine (Xylasine 2%, Rompun) diluted in 0.9% NaCl. Mice were then placed on a heated pad at 37°C. Eyes were dilated with Neosyhephrine (Neosynephrine Faure 10%, Europhta) and Mydriaticum (Mydriaticum 0.5%, Thea) diluted in 0.9% NaCl. Eyes were protected with Lubrithal eye gel before putting electrodes on the corneal surface of each eye. The reference electrode was inserted under the skin into the forehead and a ground electrode under the skin in the back.

ERG recordings were done under two conditions: (i) photopic condition, which reflects con-driven light responses - 6ms light flashes were applied every second during 60 seconds at increasing light intensities (0.1/1/10/50cd s/m) after an adaptation of 5 minutes at 20cd s/m - and (ii) flicker condition, which are rapid frequency light stimuli that reflect cone function (70 flashes at 10Hz et 1cd s/m).

Graph and statistical analysis were performed using GraphPad.

### 5. Optokinetic test

Visual acuity was measured using an optokinetic test scoring the head turning movement of a mouse placed in front of moving bars. Testing was performed using a computer-based machine consisting of four computer monitors arranged in a square to form an optokinetic chamber. A computer program was designated to generate the optokinetic stimuli, consisting of moving alternate black and white stripes. The spatial frequency is ranging from 0.03 to 0.6 cyc/deg. The program enabled modulation of stripe width and direction of bar movement.

### 6. Immunohistochemistry and confocal imaging

Animals were sacrificed by CO₂ inhalation, and the eyes were enucleated and fixed in 4% paraformaldehyde-PBS for 1h at room temperature. The eyes were dissected either as eyecups for immunohistochemistry or prepared as flat mounts for cell counting. The eyecups were then cryoprotected with a gradient of PBS-Sucrose 10% for 1h and then in PBS-Sucrose 30% overnight. The eyecups were embedded in OCT and 12 µm thick cryostat sections (ThermoFisher) were cut and mounted on glass slides. The sections were washed in PBS (3x5 mins) and stained against different antibodies (see table below) and DAPI (1:2000). The sections were finally washed in PBS, mounted in Fluoromount Vactashield (Vector Laboratories) and coverslipped for imaging using laser-confocal microscopy (Olympus IX81). For flat-mount retina stainings, the protocol is the same except that the tissue was not cryoprotected. Images were analysed using FIJI software.

| Antibody table | | |
|---|---|---|
| Target | Host | Clonality/Conjugated |
| PRIMARY ANTIBODIES | | |
| Red/Green opsin (M/L opsin) | Rabbit | Polyclonal |
| Mouse Cone arrestin | Rabbit | Polyclonal |
| PDE6C | Rabbit | Polyclonal |
| GNAT2 | Rabbit | Polyclonal |
| PNA-Lectin | Conjugated with FITC | |

| SECONDARY ANTIBODIES | | |
|---|---|---|
| Anti-rabbit | Donkey | AF 546 |

### 7. Cell counts

Flat mount retinas of rd10 and huRhoP347S^{+/-} mice were stained using antibodies against mouse cone arrestin - mCAR (1:10000) and DAPI (1:2000). The double stained cells counted at different ages. Retinas from 5 animals (n=10) were used for each age and were oriented dorso-ventrally and naso-temporally. Serial optical sections were obtained to cover the thickness of the entire outer nuclear layer (ONL). Two scanning areas of 211.97 x 211.97 µm were made in each of the four regions in all retinas. Counts of cone cells were performed manually using the FIJI software by the reconstruction of the images (z stack) covering the entire thickness of the ONL. Average density values of each retina were calculated to obtain the number of cone cells per mm² at different ages.

### 8. In vitro test of the efficiency of mouse GIRK2

HEK cells were transfected with two plasmids: CMV-SWO-mCherry and CMV-GIRK2-GFP (Figure 3) according to a well-known procedure in the art. HEK293 cells were cultured and recorded in dark room conditions after transfection. Cells were placed in the recording chamber of a microscope equipped with a 25x water immersion objective (XLPIanN-25 x -W-MP/NA1.05, Olympus) at 36 °C in oxygenated (95% 02/5% CO2) Ames medium (Sigma-Aldrich) enriched with an addition of 1 mM9-cis-retinal. KGluconate was added to the external solution in order to get a high extracellular potassium concentration leading to a cell potassium reversal potential of -40mV.

For Whole-cell recordings, the Axon Multiclamp 700B amplifier (Molecular Device Cellular Neurosciences) was used, GIRK-mediated K+-currents were recorded in voltage-clamp configuration at -80 mV, using borosilicate glass pipettes (BF100-50-10, Sutter Instrument) pulled to 5MΩ and filled with 115 mMK Gluconate, 10 mM KCI, 1 mM MgCl2, 0.5 mM CaCl2, 1.5 mM EGTA, 10 mM HEPES, and 4 mM ATP-Na2 (pH 7.2).

During experiments a CCD camera (Hamamatsu Corp.) was used to visualize cells using a trans-illuminated infrared-light. A monochromatic light source (Polychrome V, TILL photonics) was used to stimulate cells during electrophysiological experiments with light flashes at 400 nm.

### EXAMPLE 2: RESULTS

### 1. The changes in the phototransduction cascade in degenerating cones

The phototransduction cascade was first analysed in the rd10 mouse model by studying its components using immunohistochemistry, at different time points during retinal degeneration. Immunofluorescence staining was performed against cone opsin, transducing, phosphodiesterase and cone arrestin proteins of the phototransduction cascade that interact directly with cone opsin.

Figure 4 shows that only the cone opsin and arrestin were still expressed and localized around the cone cell body at late stage of the disease.

### 2. Cone opsin and GIRK2-mediated vision restoration

Based on immunohistochemistry and previous findings with cone opsins expressed in neurons, it was first studied why delivering a mouse short wavelength cone opsin (SWO) fused with tdTomato and GIRK2 fused with GFP using two AAV vectors mixed in equimolar ratios would enhance cone cell's response to light. Thus two AAVs were injected subretinally to degenerating rd10 mouse retinas at p15 (Figure 5A). This led to a significant increase in phototpic ERG amplitudes in treated eyes compared to controls (Figure 5B). Flicker ERGs confirmed that the recovery mechanism was still active in these cone cells expressing GIRK2 allowing them to follow a fast stimulus (Figure 5C). The rd10 animals treated with GIRK2 showed also an improved optokinetic reflex compared to controls (Figure 5D).

Next it was studied if the endogenous cone opsin, still present in degenerating cones, was functional and sufficient to activate GIRK2 channel in this mouse model. For this, a single AAV8 vector encoding GIRK2 in fusion with GFP was delivered. This led to similar increases in photopic ERG amplitudes and optokinetic reflex in treated eyes compared to controls confirming that GIRK2 alone was sufficient to increase light sensitivity via G protein coupled signalling involving cone opsin (Figure 6A-B). Flicker ERGs were also robustly amplified with this approach (Figure 6C).

### 3. GIRK2-mediated vision restoration: long-term efficacy

Photopic ERG recordings were performed to monitor the cone response to light stimuli at different time points after treatment with GIRK2 and in absence of treatment. These ERGs were done under two conditions: (i) photopic with light flashes applied every second during 60 seconds at increasing light intensities and (ii) flicker stimulation with repetitive flashes during 60 seconds. Data were collected on a weekly basis until p50 and then every 10 to 13 days until 11 weeks of age and showed a gradual decline in ERG amplitudes for both controls and treated eyes (Figure 7A). Moreover, these results are consistent with the optokinetic test, both controls and treated eyes with GIRK2 show a decreased optokinetic reflex over time (Figure 7B). This decline was to be expected as cone numbers also decreased over time in the rd10 mice (Figure 7C). The number of cone photoreceptors remaining in rd 10 retinas were counted to correlate decreases in cone numbers with decrease in ERG amplitudes. Indeed, the decrease in light responses was proportional to number of remaining photoreceptors (Figure 7D). It was thus concluded that GIRK2 increased light responses in remaining cones so long as cones remained alive but, as expected, it did not slow down the loss of cone cells.

### 4. GIRK2-mediated vision restoration in an RCD model caused by mutant rhodopsin

Having in mind the goal of creating a mutation-independent therapy, the approach was tested in another mouse model - with a different causal mutation. To this aim experiments were done in a heterozygous mouse model called mRho^{-/-}huRhoP347S^{+/-} carrying a knock in for P347S mutant human rhodopsin. Mutant human rhodopsin and absence of mouse rhodopsin led to a rod-cone dystrophy in this complementary model. Here, the same set of experiments was repeated as that was done in the rd10 mouse model. First, the phototransduction cascade proteins interacting with cone opsin at different time points was analysed (Figure 8). It was noticed that: (i) the degeneration rate was slower compared to rd10 and (ii) similar to the rd10 model only the opsin and the arrestin persisted in cone cell bodies at P150.

Next, mice were injected at P15 with the same AAV vectors encoding for GIRK2 fused with GFP and recorded ERGs to monitor cone response to light stimuli at various time points (Figure 9A). The response amplitudes of treated eyes were significantly higher than that of control eyes until P100. Moreover, flicker ERG responses were also similarly improved in this mouse model. Similarly to rd10 mice, this mouse model also shows an improved optokinetic reflex that decreases over time in both control and treated conditions (Figure 9B). This decline is to be expected as cone numbers also decreases over time in this RCD mouse model (Figure 9C). The decrease in time in ERG amplitudes also correlated with a decrease in cone numbers in this model (Figure 9D). This was again consistent with the fact that the approach did not stop the degeneration but allowed for enhanced light sensitivity via GIRK2.

### 5. Efficiency of the mouse GIRK2 in an in vitro test

Light stimulations (400nm, 5 seconds, fullfield) activated GIRK currents in HEK cells expressing both GIRK and SWO (Short Wavelength Opsin) (Figure 10). GIRK channels are modulated in a membrane-delimited, fast manner via the Gi/o pathway and the expression of the mouse GIRK channel was membrane bound. The amplitudes and kinetics of light-induced activation and deactivation of GIRK channels with SWO, induce large GIRK current amplitudes during a 5 s light pulse.

### List of references

1. Sinha R, Hoon M, Baudin J, Okawa H, Wong ROL, Rieke F. 2017. Cellular and Circuit Mechanisms Shaping the Perceptual Properties of the Primate Fovea. Cell. 168(3):413-426.e12
2. Yau KW, Hardie RC. 2009. Phototransduction motifs and variations. Cell. 139(2):246-64
3. Ebrey T, Koutalos Y. 2001. Vertebrate photoreceptors. Prog Retin Eye Res. 20(1):49-94
4. Larhammar D, Nordström K, Larsson T a. 2009. Evolution of vertebrate rod and cone phototransduction genes. Philosophical transactions of the Royal Society of London. Series B, Biological sciences. 364(1531):2867-80
5. Maeda T, Imanishi Y, Palczewski K. 2003. Rhodopsin phosphorylation: 30 Years later. Prog. Retin Eye Res. 22(4): 417-434
6. Buch H, Vinding T, La Cour M, Appleyard M, Jensen GB, Nielsen NV. 2004. Prevalence and Causes of Visual Impairment and Blindness among 9980 Scandinavian Adults: The Copenhagen City Eye Study. Ophthalmology. 111(1):53-61
7. Wright AF, Chakarova CF, Abd El-Aziz MM, Bhattacharya SS. 2010. Photoreceptor degeneration: genetic and mechanistic dissection of a complex trait. Nature reviews. Genetics. 11(4):273-84
8. Ferrari S, Di lorio E, Barbaro V, Ponzin D, Sorrentino FS, Parmeggiani F. 2011. Retinitis pigmentosa: genes and disease mechanisms. Current genomics. 12(4):238-49
9. Li ZY, Kljavin IJ, Milam a H. 1995. Rod photoreceptor neurite sprouting in retinitis pigmentosa. The Journal of neuroscience: the official journal of the Society for Neuroscience. 15(8):5429-38
10. Bennett J. 2017. Taking Stock of Retinal Gene Therapy: Looking Back and Moving Forward. Molecular Therapy. 25(5):1076-94
11. Dalkara D, Duebel J, Sahel J-A. 2015. Gene therapy for the eye focus on mutation-independent approaches. Current Opinion in Neurology. 28(1):51-60
12. Busskamp V, Picaud S, Sahel JA, Roska B. 2012. Optogenetic therapy for retinitis pigmentosa. Gene Therapy. 19(2):169-75
13. Dalkara D, Sahel J-A. 2014. Gene therapy for inherited retinal degenerations. C. R. Biol. 337(3): 185-192
14. Scholl HPN, Strauss RW, Singh MS, Dalkara D, Roska B, et al. 2016. Emerging therapies for inherited retinal degeneration. Science Translational Medicine. 8(368):368rv6-368rv6
15. Baker CK, Flannery JG. 2018. Innovative Optogenetic Strategies for Vision Restoration. Frontiers in Cellular Neuroscience. 12(September):1-8
16. Cehajic-Kapetanovic J, Eleftheriou C, Allen AE, Milosavljevic N, Pienaar A, et al. 2015. Restoration of Vision with Ectopic Expression of Human Rod Opsin. Current Biology. 25(16):2111-22
17. Gaub BM, Berry MH, Holt AE, Isacoff EY, Flannery JG. 2015. Optogenetic Vision Restoration Using Rhodopsin for Enhanced Sensitivity. Molecular therapy: the journal of the American Society of Gene Therapy. 23(10):1562-71
18. Van Gelder RN, Kaur K. 2015. Vision Science: Can Rhodopsin Cure Blindness. Current Biology. 25(16):R713-15
19. van Wyk M, Pielecka-Fortuna J, Löwel S, Kleinlogel S. 2015. Restoring the ON Switch in Blind Retinas: Opto-mGluR6, a Next-Generation, Cell-Tailored Optogenetic Tool. PLOS Biology. 13(5):e1002143
20. Berry MH, Holt A, Levitz J, Visel M, Aghi K, et al. Restoration of high-sensitivity , adapting , patterned vision with a cone opsin. Nature Communications. (2019):1-12
21. De Silva SR, Barnard AR, Hughes S, Tam SKE, Martin C, et al. 2017. Long-term restoration of visual function in end-stage retinal degeneration using subretinal human melanopsin gene therapy. Proceedings of the National Academy of Sciences. 114(42):11211-16
22. Lin B, Koizumi A, Tanaka N, Panda S, Masland RH. 2008. Restoration of visual function in retinal degeneration mice by ectopic expression of melanopsin. Proceedings of the National Academy of Sciences of the United States of America. 105(41):16009-14
23. Masseck OA, Spoida K, Dalkara D, Maejima T, Rubelowski JM, et al. 2014. Vertebrate Cone Opsins Enable Sustained and Highly Sensitive Rapid Control of G i/o Signaling in Anxiety Circuitry. Neuron. 81(6):1263-73
24. Mark MD, Herlitze S. 2000. G-protein mediated gating of inward-rectifier K+ channels. Eur. J. Biochem. 267(19): 5830-5836
25. Busskamp V, Duebel J, Balya D, Fradot M, Viney TJ, et al. 2010. Genetic Reactivation of Cone Photoreceptors Restores Visual Responses in Retinitis Pigmentosa. Science. 329(5990):413-17
26. Packer AM, Roska B, Häusser M. 2013. Targeting neurons and photons for optogenetics. Nature neuroscience. 16(7):805-15
27. Khabou H, Garita-Hernandez M, Chaffiol A, Reichman S, Jaillard C, et al. 2018. Noninvasive gene delivery to foveal cones for vision restoration. JCI Insight. 3(2): 1-18
28. Demande internationale WO 2018134168
29. Byrne LC, Dalkara D, Luna G, Fisher SK, Clérin E, et al. 2015. Viral-mediated RdCVF and RdCVFL expression protects cone and rod photoreceptors in retinal degeneration. Journal of Clinical Investigation. 125(1):105-16
30. Millington-Ward S, Chadderton N, O'Reilly M, Palfi A, Goldmann T, et al. 2011. Suppression and Replacement Gene Therapy for Autosomal Dominant Disease in a Murine Model of Dominant Retinitis Pigmentosa. Molecular Therapy. 19(4):642-49

## Claims

1. Vector comprising a nucleotide sequence encoding subunit 2 of G-protein-gated inwardly rectifying potassium channel (GIRK2) or a functional derivative thereof.

2. Vector according to claim 1, wherein the nucleotide sequence encoding GIRK2 or a derivative thereof comprises the sequence SEQ ID NO:1, SEQ ID NO:3 or SEQ ID NO:5.

3. Vector according to claim 1 or 2, further comprising a nucleotide sequence encoding a mammal cone opsin.

4. Carrier including the vector defined in any one of claims 1 to 3.

5. Carrier including the vector according to claim 1 or 2 and further including a vector comprising a nucleotide sequence encoding a mammal cone opsin.

6. Carrier according to claim 4 or 5, wherein the carrier is chosen from the group consisting of solid-lipid nanoparticles, chitosan nanoparticles, liposome, lipoplex and cationic polymer.

7. Vector according to claim 3 or carrier according to any one of claims 4 to 6, wherein the mammal cone opsin is a short wavelength cone opsin (SWO).

8. Vector according to any one of claims 1 to 3 and 7 or carrier according to any one of claims 4 to 6, wherein the vector is chosen from the group consisting of an adeno-associated viral (AAV), an adenovirus, a lentivirus, SV40 viral vector.

9. Pharmaceutical composition comprising the vector according to any one of claims 1 to 3 and 7, or the carrier according to any one of claims 4 to 6, with a pharmaceutically acceptable carrier, diluent or excipient.

10. Vector according to any one of claims 1 or 3 and 7, carrier according to any one of claims 4 to 6 or pharmaceutical composition according to claim 9, for use in treating rod-cone dystrophy (RCD).

11. Nucleotide sequence encoding subunit 2 of G-protein-gated inwardly rectifying potassium channel (GIRK2) or a functional derivative thereof, for use as a medicament.

12. Nucleotide sequence encoding subunit 2 of G-protein-gated inwardly rectifying potassium channel (GIRK2) or a functional derivative thereof, for use in treating rod-cone dystrophy (RCD).

13. Nucleotide sequence for use according to claim 11 or 12, wherein the nucleotide sequence comprises the sequence SEQ ID NO:1, SEQ ID NO:3 or SEQ ID NO:5.
